(19) Europäisches Patentamt
European Patent Office
Office européen des brevets

(11) Veröffentlichungsnummer: **0 093 252**
**B1**

(12) # EUROPÄISCHE PATENTSCHRIFT

(45) Veröffentlichungstag der Patentschrift: **09.03.88**

(21) Anmeldenummer: **83102598.6**

(22) Anmeldetag: **16.03.83**

(51) Int. Cl.[4]: **C 07 D 401/12**, C 07 D 213/70, C 07 D 213/32, C 07 D 413/12, C 07 D 498/04, C 07 D 487/04, A 61 K 31/325 // (C07D498/04, 263:00, 221:00),(C07D487/04, 239:00, 231:00)

(54) **Thiomethylpyridin-Derivate, Verfahren zu ihrer Herstellung und diese Verbindungen enthaltende Arzneimittel.**

(30) Priorität: **05.05.82 DE 3216843**

(43) Veröffentlichungstag der Anmeldung: **09.11.83 Patentblatt 83/45**

(45) Bekanntmachung des Hinweises auf die Patenterteilung: **09.03.88 Patentblatt 88/10**

(84) Benannte Vertragsstaaten: **AT BE CH DE FR GB IT LI LU NL SE**

(56) Entgegenhaltungen:
**EP - A - 0 005 129**
**EP - A - 0 074 341**
**DE - A - 1 804 450**
**DE - A - 1 906 952**
**FR - A - 2 348 201**
**FR - A - 2 392 021**
**GB - A - 1 406 882**
**GB - A - 1 434 405**
**GB - A - 2 014 560**
**GB - A - 2 038 825**
**GB - A - 2 069 493**
**GB - A - 2 082 580**
**NL - A - 7 501 917**

**JOURNAL OF THE CHEMICAL SOCIETY, PERKIN TRANSACTIONS 1, Nr. 2, Seiten 418-425, London, GB,**

(73) Patentinhaber: **LUDWIG HEUMANN & CO GMBH, Heideloffstrasse 18-28 Postfach 2260, D-8500 Nürnberg (DE)**

(72) Erfinder: **Schickaneder, Helmut, Dr., Dipl.-Chem., Moosäcker 25, D-8501 Eckental (DE)**
Erfinder: **Engler Hintermayer, Heidrun, Dr., Ringstrasse 23, D-8501 Cadolzburg (DE)**
Erfinder: **Szelenyi, Istvan, Dr., Brahmsstrasse 16, D-8501 Schwaig (DE)**

(74) Vertreter: **Kraus, Walter, Dr. et al, Patentanwälte Kraus, Weisert & Partner Thomas-Wimmer-Ring 15, D-8000 München 22 (DE)**

(56) Entgegenhaltungen: (Fortsetzung)
**A.R. KATRITZKY u.a.: "Heterocycles in organic synthesis. Part 6. Nucleophilic displacements of primary amino-groups via 2,4,6-triphenylpyridinium salts"**
**CHEMICAL ABSTRACTS, vol. 98, 1983, Seite 20, Nr. 46446b, Columbus, Ohio, US, F. HAVIV u.a.: "2-[(Phenylthio)methyl]pyridine derivatives: new antiinflammatory agents"**

**Die Akte enthält technische Angaben, die nach dem Eingang der Anmeldung eingereicht wurden und die nicht in dieser Patentschrift enthalten sind.**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft neue Thiomethylpyridin-Derivate, ein Verfahren zu ihrer Herstellung und diese Verbindungen enthaltende Arzneimittel.

Die erfindungsgemässen neuen Verbindungen zeichnen sich überraschenderweise durch eine hohe bronchosekretolytische und mukolytische Wirkung aus, die therapeutisch nutzbar ist.

Bekanntlich ist die Viskositätsverringerung des Sputums durch Arzneimittel bei der Behandlung von akuten und chronischen Bronchialerkrankungen (zum Beispiel Infektionen der Atemwege, asthmatischen Beschwerden, obstruktiven Erkrankungen u.dgl.) ein wichtiges therapeutisches Ziel. Für diese Krankheitsbilder sind Arzneimittel unterschiedlicher Art bekannt. Sie unterscheiden sich durch ihre lokale bzw. systemische Wirksamkeit. Zur lokal wirksamen Arzneimittelgruppe zählt u.a. N-Acetylcystein. Zu den systemisch wirksamen Vertretern – und in der Therapie der Bronchialerkrankungen etabliertes Bronchosekretolytikum – gehört zum Beispiel Ambroxol, d.h. trans-4-(2-Amino-3,5-dibrombenzyl)-aminocyclohexanol. Diese Verbindung wird beispielsweise in der DE-PS 1 593 579 beschrieben.

Aufgabe der Erfindung ist es, neue bronchosekretolytisch und mukolytisch wirkende Verbindungen zur Verfügung zu stellen, deren orale und parenterale Wirksamkeit gegenüber anerkannt guten Wirkstoffen gleicher Wirkungsrichtung, wie zum Beispiel Ambroxol, wesentlich verbessert ist.

Diese Aufgabe wird durch die Erfindung gelöst.

Gegenstand der Erfindung sind daher neue Thiomethylpyridin-Derivate der allgemeinen Formel I

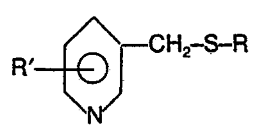

in der R die Gruppe 2-Pyrimidinyl, 2-(4-Methylpyrimidinyl), 2-(4,6-Dimethylpyrimidinyl), 2-Benzoxazolyl, 6-Methyl-2-benzoxazolyl, 2-Oxazolo-(4,5-c)-pyridinyl, 4-Tolyl, 4-Chlorphenyl, 2-(1-Methylimidazolyl), 2-(4,5-Diphenyloxazolyl) oder 4-(1H-Pyrazolo-(3,4-d)-pyrimidinyl) ist und R′ für ein Wasserstoffatom oder einen in 2- oder 6-Position angeordneten Methyl- oder Chlorrest steht, sowie ihre therapeutisch verträglichen Säureadditionssalze.

Die Verbindungen der allgemeinen Formel I bilden leicht Säureadditionssalze, zum Beispiel Mono-, Di- und Triadditionssalze, wie beispielsweise Hydrochloride, Hydrobromide, Sulfate, Acetate, Maleate, Fumarate, Oxalate, Succinate und Embonate etc.

Die erfindungsgemässen Verbindungen können durch ein Verfahren hergestellt werden, das dadurch gekennzeichnet ist, dass man in an sich bekannter Weise ein Thiolat der allgemeinen Formel II

R–SMe (II)

in der R die oben angegebene Bedeutung hat und Me für ein Alkaliatom steht, mit einem Picolylchlorid-Hydrohalogenid der Formel

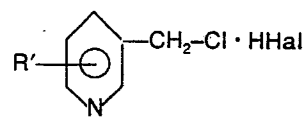

worin R′ wie oben definiert ist und Hal ein Halogenatom bedeutet, in der wässrig-alkoholischen Alkalihydroxidlösung, wobei das Alkalihydroxid im Überschuss über die stöchiometrische Menge vorliegt, umsetzt und gegebenenfalls die so erhaltene freie Base in ein therapeutisch verträgliches Säureadditionssalz umwandelt.

In der Formel II bedeutet Me ein Alkaliatom, beispielsweise ein Kalium- oder Natriumatom, wobei ein Natriumatom bevorzugt wird. In der allgemeinen Formel IV bedeutet Hal ein Halogenatom, beispielsweise ein Chlor- oder Bromatom, vorzugsweise ein Chloratom. Die Umsetzung findet in einer wässrig-alkoholischen Alkalihydroxidlösung statt, wobei das Alkalihydroxid im Überschuss über die stöchiometrische Menge, vorzugsweise in mindestens der doppelten äquimolaren Menge, vorliegt. Als wässrig-alkoholische Lösung kommt vorzugsweise eine wässrig-ethanolische Lösung in Betracht. Als Alkalihydroxid wird Natriumhydroxid bevorzugt. Bei der Umsetzung geht man zweckmässig so vor, dass man das Thiolat der allgemeinen Formel II bei 0 bis 5 °C in der wässrig-alkoholischen Alkalihydroxidlösung auflöst und anschliessend mit der äquimolaren Menge der Picolylchlorid-Hydrohalogenidverbindung, gelöst in wässrig-alkoholischer, vorzugsweise wässrig-ethanolischer, Lösung, 2 bis 6 Stunden, vorzugsweise 4 Stunden, bei Raumtemperatur zur Umsetzung bringt.

Aufgrund des angewendeten stöchiometrischen Überschusses des Alkalihydroxids wird die freie Base erhalten, die sodann in üblicher Weise durch Umsetzung mit einer pharmazeutisch annehmbaren Säure in ein therapeutisch annehmbares Salz umgewandelt werden kann.

Die erfindungsgemässen Verbindungen zeichnen sich durch eine überraschend verbesserte bronchosekretolytische und mukolytische Wirkung aus, so dass sie für die Therapie in wesentlich kleineren Mengen als bekannte Wirkstoffe dosiert werden können.

Ein weiterer Gegenstand der Erfindung ist daher eine pharmazeutische Zubereitung mit bronchosekretolytischer und mukolytischer Wirkung, die bzw. das neben üblichen Hilfs- und Trägerstoffen wenigstens ein Thiomethylpyridin-Derivat der allgemeinen Formel I enthält.

Die erfindungsgemässe pharmazeutische Zubereitung kann für alle Arten von Bronchialerkrankungen, beispielsweise akute und chronische Atemwegserkrankungen, zur Nachbehandlung von chirurgischen Eingriffen in den Atemwegen sowie bei allen Prozessen, bei denen eine Verflüssigung des Bronchialschleims wünschenswert ist, verwendet werden.

Die erfindungsgemäss verwendete Verbindung wird vorzugsweise oral verabreicht. Gewöhnlich beträgt die orale Tagesdosis 0,01 bis 0,2 g, vorzugsweise 0,02 bis 0,1 g, die in einer oder mehreren Tagesdoses verabreicht werden kann. Im Einzelfall kann es gegebenenfalls erforderlich sein, von den genannten Mengen abzuweichen, und zwar in Abhängigkeit vom individuellen Verhalten gegenüber dem Wirkstoff bzw. der Art seiner Formulierung und dem Zeitpunkt bzw. Intervall, zu welchem die Verabreichung erfolgt. So gibt es zum Beispiel Fälle, wo mit weniger als der oben genannten Mindestmenge ausgekommen werden kann, während in anderen Fällen die genannte obere Grenze überschritten werden muss. Im Falle der Applikation grösserer Mengen kann es empfehlenswert sein, diese in mehreren Einzelgaben über den Tag zu verteilen.

Für die orale Verabreichung kann der Wirkstoff beispielsweise in Form von Kapseln formuliert werden, die durch herkömmliche Massnahmen mit pharmazeutisch annehmbaren Exzipientien, z.B. Bindemitteln (wie vorgelatinisierter Maisstärke, Polyvinylpyrrolidon oder Hydroxypropylmethylcellulose); Füllstoffen (wie Lactose, mikrokristalline Cellulose oder Calciumphosphat); Schmiermitteln (wie Magnesiumstearat, Talk oder Kieselsäure); Sprengmitteln (z.B. Kartoffelstärke oder Natriumstärkeglykollat); oder Befeuchtungsmitteln (z.B. Natriumlaurylsulfat), hergestellt werden. Die Kapseln können nach bekannten Methoden beschichtet werden. Flüssige Zubereitungen für die orale Verabreichung oder für ein direktes Einträufeln können beispielsweise die Form von Lösungen, Sirups oder Suspensionen haben, oder sie können als Trockenprodukt zur Rekonstitution mit Wasser oder einem anderen geeigneten Träger vor dem Gebrauch präsentiert werden. Solche flüssigen Zubereitungen können durch herkömmliche Massnahmen mit pharmazeutisch annehmbaren Additiven, z.B. Suspendierungsmitteln (wie Sorbitsirup, Methylcellulose oder hydrierten essbaren Fetten); Emulgierungsmitteln (z.B. Lecithin oder Acacia); nichtwässrigen Trägern (z.B. Mandelöl, ölige Ester oder Ethylalkohol); und Konservierungsmitteln (z.B. Methyl- oder Propyl-p-hydroxybenzoaten oder Sorbinsäure), hergestellt werden.

Für die bukkale Verabreichung können die Zubereitungen in Form von Tabletten oder Lutschpastillen, die auf herkömmliche Weise formuliert werden, vorliegen.

Die erfindungsgemäss verwendete Verbindung kann für die parenterale Verabreichung durch Injektion oder für die Infusion formuliert werden. Zubereitungen für die Injektion können in Einheitsdosisform, z.B. in Ampullen oder in Viel-Dosen-Behältern, mit einem zugegebenen Konservierungsstoff vorliegen. Die Zubereitungen können auch solche Formen, wie Suspensionen, Lösungen oder Emulsionen in öligen oder wässrigen Trägern einnehmen, und sie können Formulierungsmittel, wie Suspendierungs-, Stabilisierungs- und/oder Dispergierungsmittel, enthalten. Alternativ kann der Wirkstoff auch in Pulverform zur Rekonstitution mit einem geeigneten Träger, z.B. sterilem, pyrogenfreiem Wasser, vor dem Gebrauch vorliegen.

Zur Verabreichung durch Inhalierung wird die erfindungsgemäss verwendete Verbindung geeigneterweise in Form eines Aerosolsprays aus unter Druck gesetzten Packungen oder Zerstäubern unter Verwendung eines geeigneten Treibmittels, z.B. Dichlordifluormethan, Trichlorfluormethan, Dichlortetrafluorethan, Kohlendioxid oder eines anderen geeigneten Gases, abgegeben. Im Falle eines unter Druck gesetzten Aerosols kann die Dosiseinheit in der Weise bestimmt werden, dass ein Ventil zur Abgabe einer dosierten Menge vorgesehen ist.

Pharmakologische Untersuchungen haben ergeben, dass die erfindungsgemäss verwendeten Thiomethylpyridin-Derivate gegenüber dem bekannten Vergleichsprodukt Ambroxol überlegene bronchosekretolytische und mukolytische Eigenschaften aufweisen. Im einzelnen wurden folgende Untersuchungen durchgeführt:

1. Pharmakodynamik

1.1. Sekretstimulierende Aktivität

Bronchosekretolytisch wirksame Verbindungen fördern die tracheale Ausscheidung von Phenolrot (Chronic Bronchitis Research Group, Chinese Medical Journal 3 : 259, 1977). Die Steigerung der trachealen Phenolrot-Ausscheidung ist ein Mass der bronchosekretolytischen Wirkung. Die diesbezügliche Wirkung der Prüfsubstanzen wurde nach oraler Verabreichung an wachen Mäusen untersucht. Die in der Tabelle I angegebenen $ED_{50}$-Werte wurden anhand der Regressionskurven berechnet. Wie aus der Tabelle ersichtlich ist, war 2-(Pyridyl-3-methylthio)-pyrimidin-Hydrochlorid am stärksten wirksam. Seine die Phenolrot-Ausscheidung fördernde Wirkung war etwa 160 mal stärker als die des bekannten Bronchosekretolytikums, Ambroxol.

Tabelle I

| Verbindung | $ED_{50}$ (mg/kg) |
|---|---|
| Ambroxol | 250 |
| Beispiel 1 | 1,5 |
| Beispiel 2 | 10 |
| Beispiel 3 | 12 |
| Beispiel 4 | 15 |
| Beispiel 5 | 15 |
| Beispiel 6 | 12 |
| Beispiel 7 | 10 |
| Beispiel 8 | 6 |

1.2. Muko-Sekretolytische Aktivität beim Hund

An narkotisierten mischrassigen Hunden wurde bronchialer Schleim unmittelbar vor und eine Stunde nach intravenöser Gabe der Prüfsubstanzen mit Hilfe eines Bronchoskops entnommen und in Phosphatpuffer aufgenommen. Nach Dialysierung und Gefriertrocknung wurden die Schleimproben in 0,1M Tris-Puffer (pH 7) aufgenommen

(Endkonzentration 1% w/v). Die Messung der Viskosität erfolgte im Rotationsviskosimeter (Fa. Contraves, Stuttgart).

Wie aus der Tabelle II ersichtlich ist, bewirkte das bekannte Bronchosekretolytikum Ambroxol eine deutliche Abnahme der Viskosität des bronchialen Schleims. Die auf Gewichtsbasis bezogene identische Menge von 2-(Pyridyl-3-methylthio)-pyrimidin-Hydrochlorid senkte die Viskosität des bronchialen Schleims noch deutlicher.

Tabelle II

| Verbindung | Dosis und Applikationsart | Viskosität mPa × s<br>Vorwert | 1 Stunde nach Substanzgabe |
|---|---|---|---|
| Kontrolle<br>(0,9% NaCl) | i.v. | 4,2<br>(3,6–4,8) | 4,8<br>(4,4–5,2) |
| Beispiel 1 | 10 mg/kg i.v. | 3,9<br>(3,2–4,5) | 1,2<br>(1,0–1,4) |
| Ambroxol | 10 mg/kg i.v. | 3,8<br>(3,6–4,0) | 2,3<br>(1,4–3,2) |

2. Toxikologische Ergebnisse

Die erfindungsgemäss beanspruchten Verbindungen zeigen in akuten Versuchen eine niedrige orale Toxizität. So fand sich ein $LD_{50}$-Wert für 2-(Pyridyl-3-methylthio)-pyrimidin-Hydrochlorid von 1156 mg/kg p.o. bei Mäusen. Unter identischen Bedingungen erwies sich Ambroxol als etwas weniger toxisch ($LD_{50}$: 2720 mg/kg p.o. nach Püschmann et al. Arzn. Forsch. 28 : 889, 1978).

Die Erfindung wird in den Beispielen erläutert.

Beispiel 1

Herstellung von 2-(Pyridyl-3-methylthio)-pyrimidin-Hydrochlorid

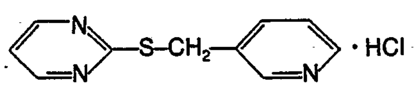

Zu einer Lösung aus 11,2 g (0,1 mol) 2-Mercapto-pyrimidin in 250 ml Ethanol werden bei 0 °C 8,4 g (0,21 mol) Natriumhydroxid, gelöst in 120 ml Wasser, zugetropft. Anschliessend werden 16,4 g (0,1 mol) 3-Picolylchlorid-Hydrochlorid, gelöst in 100 ml Wasser, langsam zugegeben und 4 Stunden bei Raumtemperatur gerührt. Die Reaktionslösung wird eingeengt, in 500 ml Ether aufgenommen, die organische Phase dreimal mit je 100 ml Wasser gewaschen, über Natriumsulfat getrocknet, abfiltriert und das Filtrat im Vakuum eingeengt. Der anfallende Feststoff, den die Base darstellt, wird aus Hexan umkristallisiert.

Farblose Kristalle vom Schmelzpunkt 53–54 °C; $R_f$ = 0,5 ($CH_2Cl_2$/MeOH 9:1); Ausbeute 9,14 g (45%)

$C_{10}H_9N_3S$ (203) Berechnet:
C 59,09 H 4,46 N 20,67 S 15,77
Gefunden:
C 59,20 H 4,47 N 20,65 S 15,73

$^1$H-NMR-Spektrum
($CDCl_3$): δ = 4,40 (s) ($-SCH_2-$) 2 H, 7,0 (t) (Aromaten-H) 1 H, 7,20 (m) (Aromaten-H) 1 H, 7,80 (d) (Aromaten-H) 1 H, 8,53 (m) (Aromaten-H) 3 H, 8,73 (s) (Aromaten-H) 1 H ppm

Die Herstellung des Hydrochlorids erfolgt durch Versetzen einer etherischen Lösung aus 2-(Pyridyl-3-methylthio)-pyrimidin mit einer äquimolaren 10%igen ethanolischen HCl-Lösung. Dabei fällt das Hydrochlorid analysenrein an.

Farblose Kristalle vom Schmelzpunkt 134–135 °C; $R_f$ = 0,85 ($CH_2Cl_2$/MeOH, 8/2, $NH_3$-Dämpfe); Ausbeute (quant.)

$C_{10}H_{10}ClN_3S$ (240) Ber.:
C 50,10 H 4,20 N 17,53 S 13,37
Gef.:
C 50,18 H 4,10 N 17,45 S 13,36

$^1$H-NMR-Spektrum
($D_2O$): δ = 5,03 (s) ($-S-CH_2$) 2 H, 7,70 (t) (Aromaten-H) 1 H, 8,43 (m) (Aromaten-H) 1 H, 9,03 (d) (Aromaten-H) 2 H, 9,13 (t) (Aromaten-H) 2 H, 9,37 (s) (Aromaten-H) 1 H ppm

Beispiel 1a

Herstellung von 2-(Pyridyl-3-methylthio)-pyrimidin-Succinat

Die Herstellung des Succinats erfolgt durch Versetzen einer ethanolischen Lösung aus 2-(Pyridyl-3-methylthio)-pyrimidin mit einer äquimolaren ethanolischen Bernsteinsäure-Lösung. Nach Einengen der Reaktionslösung fällt das Succinat analysenrein an.

Farblose Kristalle vom Schmelzpunkt 98 °C
Rf = 0,78 ($CH_2Cl_2$/MeOH8:2 $NH_3$-Dämpfe)
Ausbeute: (quant.)

$C_{14}H_{15}N_3O_4S$ (321) Ber.:
C 52,33 H 4,71 N 13,08 S 9,98
Gef.:
C 52,31 H 4,78 N 13,06 S 9,92

$^1$H-NMR-Spektrum
($d_6$-DMSO): δ = 2,40 (s) ($-CH_2-CH_2-$) 4 H, 4,43 (s) ($S-CH_2$) 2 H, 7,27 (t) (Aromaten-H) 1 H, 7,37 (m) (Aromaten-H) 1 H, 7,87 (d) (Aromaten-H) 1 H, 8,47 (d) (Aromaten-H) 1 H, 8,67 (m) (Aromaten-H) 3 H ppm.

Beispiel 2
Herstellung von 2-(Pyridyl-3-methylthio)-4-methyl-pyrimidin-Hydrochlorid

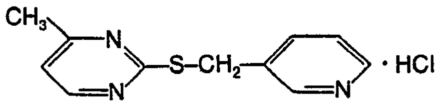

Die Herstellung erfolgt analog Beispiel 1 aus 2-Mercapto-4-methyl-pyrimidin und 3-Picolylchlorid-Hydrochlorid.
Farblose Kristalle vom Schmelzpunkt 154 °C
$R_f$ = 0,8 ($CH_2Cl_2$/MeOH, 8/2, $NH_3$-Dämpfe); Ausbeute 10,9 g (43%)
$C_{11}H_{12}ClN_3S$ (254) Ber.:
C 52,07 H 4,77 N 16,56 S 12,63
Gef.:
C 52,18 H 4,81 N 16,58 S 12,55
$^1$H-NMR-Spektrum
($D_2O$): δ = 2,80 (2) ($CH_3$) 3 H, 4,93 (s) (S–$CH_2$) 2 H, 7,43 (d) (Aromaten-H) 1 H, 8,47 (t) (Aromaten-H) 1 H, 8,73 (d) (Aromaten-H) 1 H, 9,17 (m) (Aromaten-H) 2 H, 9,47 (s) (Aromaten-H) 1 H ppm

Beispiel 3
Herstellung von 2-(Pyridyl-3-methylthio)-4,6-dimethyl-pyrimidin-Hydrochlorid

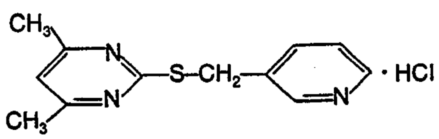

Die Herstellung erfolgt analog Beispiel 1 aus 4,6-Dimethyl-2-mercapto-pyrimidin und 3-Picolylchlorid-Hydrochlorid.
Farblose Kristalle vom Schmelzpunkt 155–156 °C
$R_f$ = 0,8 ($CH_2Cl_2$ / MeOH / 8/2 / $NH_3$-Dämpfe); Ausbeute 10,2 g (38%)
$C_{12}H_{14}ClN_3S$ (268) Ber.:
C 53,83 H 5,27 N 15,69 S 11,97
Gef.:
C 53,79 H 5,37 N 15,70 S 11,93
$^1$H-NMR-Spektrum
($D_2O$): δ = 2,73 (s) (2 × $CH_3$) 6 H, 4,90 (s) (S–$CH_2$) 2 H, 7,20 (s) (Aromaten-H), 1 H, 8,43 (t) (Aromaten-H), 1 H, 8,83 (d) (Aromaten-H), 2 H, 9,13 (s) Aromaten-H) 1 H ppm

Beispiel 4
Herstellung von 2-(Methylthio-3-pyridyl)-benzoxazol

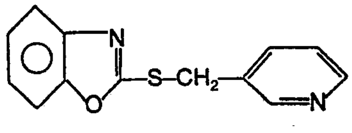

Die Herstellung erfolgt analog Beispiel 1 aus 2-Mercaptobenzoxazol und 3-Picolylchlorid-Hydrochlorid.
Farblose Kristalle vom Schmelzpunkt 88 °C
$R_f$ = 0,85 ($CH_2Cl_2$ / $CH_3OH$ / 85/15); Ausbeute 11,1 g (46%)
$C_{13}H_{10}N_2OS$ (242)
$^1$H-NMR-Spektrum
($CDCl_3$): δ = 4,5 (s) (S–$CH_2$) 2 H, 7,13–7,93 (m) (Aromaten-H) 6 H, 8,53 (d) (Aromaten-H) 1 H, 8,80 (s) (Aromaten-H) 1 H ppm

Beispiel 5
Herstellung von 6-Methyl-2-(methylthio-3-pyridyl)-benzoxazol-Hydrochlorid

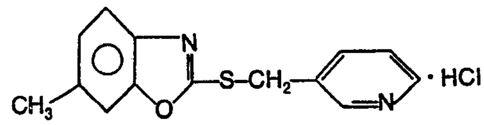

Die Herstellung erfolgt analog Beispiel 1 aus 6-Methyl-2-mercaptobenzoxazol und 3-Picolylchlorid-Hydrochlorid.
Farblose Kristalle vom Schmelzpunkt 182–183 °C
$R_f$ = 0,86 ($CH_2Cl_2$ / MeOH / 8/2 / $NH_3$-Dämpfe); Ausbeute 18,4 g (63%)
$C_{14}H_{13}ClN_2OS$ (293)
Ber.: C 57,43 H 4,48 N 9,57
Gef.: C 57,49 H 4,57 N 9,57
$^1$H-NMR-Spektrum
($D_2O$): δ = 2,53 (s) (–$CH_3$) 3 H, 5,00 (s) (S–$CH_2$) 2 H, 7,20–7,67 (m) (Aromaten-H) 3 H, 8,40 (m) (Aromaten-H) 1 H, 9,10 (m) (Aromaten-H) 2 H, 9,33 (s) (Aromaten-H) 1 H ppm

Beispiel 6
Herstellung von 2-(Methylthio-3-pyridyl)-oxazolo(4,5-c)pyridin-Dihydrochlorid

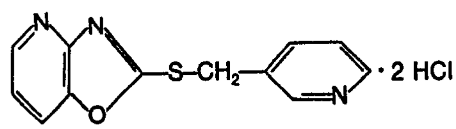

Die Herstellung erfolgt analog Beispiel 1 aus 2-Mercapto-oxazolo(4,5-c)pyridin und 3-Picolylchlorid-Hydrochlorid.
Farblose Kristalle vom Schmelzpunkt 183–185 °C
$R_f$ = 0,77 ($CH_2Cl_2$ / MeOH / 8/2 / $NH_3$-Dämpfe); Ausbeute 17,4 g (55%)
$C_{12}H_{11}Cl_2N_3OS$ (316)
Ber.: C 45,58 H 3,51 N 13,29 S 10,14
Gef.: C 45,04 H 3,52 N 12,91 S 10,05
$^1$H-NMR-Spektrum
($D_2O$): δ = 5,40 (s) (S–$CH_2$) 2 H, 8,03–9,47 (m) (Aromaten-H) 6 H, 9,60 (s) (Aromaten-H) 1 H ppm

Beispiel 7
Herstellung von 4-Tolyl-3-methylthio-pyridin-Hydrochlorid

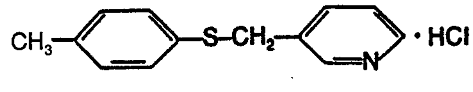

Die Herstellung erfolgt analog Beispiel 1 aus p-Methylthiophenol und 3-Picolylchlorid-Hydrochlorid.
Farblose Kristalle vom Schmelzpunkt 148–149 °C
$R_f$ = 0,71 (MeOH / $NH_3$-Dämpfe); Ausbeute 11,8 g (47%)
$C_{13}H_{14}ClNS$ (252)
Ber.: C 62,02 H 5,61 N 5,56
Gef.: C 62,08 H 5,55 N 5,61

$^1$H-NMR-Spektrum
(D₂O): δ = 2,57 (s) (–$CH_3$) 3 H, 4,67 (s) (S–$CH_2$) 2 H, 7,47 (s) (Aromaten-H) 4 H, 8,17–9,24 (m) (Aromaten-H) 4 H ppm

Beispiel 8

Herstellung von 4-Chlorphenyl-3-methylthio-pyridin-Hydrochlorid

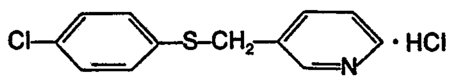

Die Herstellung erfolgt analog Beispiel 1 aus 4-Chlorthiophenol und 3-Picolylchlorid-Hydrochlorid.

Farblose Kristalle vom Schmelzpunkt 152–153 °C
$R_f$ = 0,58 (MeOH / $NH_3$-Dämpfe); Ausbeute 18,5 g (68%)
$C_{12}H_{11}Cl_2NS$ (272)
Ber.: C 52,95 H 4,07 N 5,15
Gef.: C 52,95 H 4,10 N 5,32
$^1$H-NMR-Spektrum
($D_2O$): δ = 4,73 (s) (S–$CH_2$) 2 H, 7,47 (s) (Aromaten-H) 4 H, 8,20–9,30 (m) (Aromaten-H) 4 H ppm

Beispiel 9

Herstellung von 2-(Pyridyl-6-methyl-3-methylthio)-pyrimidin-Succinat

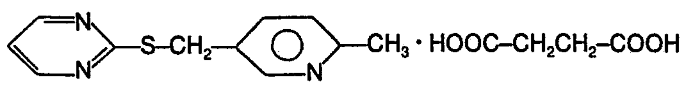

Die Herstellung erfolgt analog Beispiel 1 aus 2-Mercaptopyrimidin und 6-Methyl-3-picolylchlorid-Hydrochlorid.
Farblose Kristalle vom Schmelzpunkt 110–111 °C
Rf = 0,63 ($CH_2Cl_2$ / MeOH 95:5 $NH_3$-Dämpfe)
Ausbeute: 12,4 g (37%)
$C_{15}H_{17}N_3O_4S$ (335)
Ber.: C 53,72 H 5,11 N 12,53
Gef.: C 53,63 H 5,19 N 12,48
$^1$H-NMR-Spektrum
($d_6$-DMSO): δ = 2,43 (s) (–$CH_2$–$CH_2$–) (–$CH_3$) 7 H, 4,40 (s) (S–$CH_2$) 2 H, 7,10–7,33 (m) (Aromaten-H) 2 H, 7,77 (d) (Aromaten-H) 1 H, 8,50 (s) (Aromaten-H) 1 H, 8,67 (d) (Aromaten-H) 2 H ppm.

Beispiel 10

Herstellung von 2-(Pyridyl-2-chlor-3-methylthio) pyrimidin

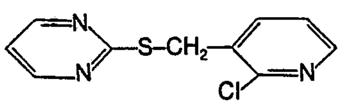

Die Herstellung erfolgt analog Beispiel 1 aus 2-Mercaptopyrimidin und 2-Chlor-3-picolylchlorid-Hydrochlorid.
Farblose Kristalle vom Schmelzpunkt 96–97 °C
Rf = 0,69 ($CH_2Cl_2$ / MeOH 9:1)
Ausbeute: 15,1 g (64%)
$C_{10}H_8ClN_3S$ (238)
$^1$H-NMR-Spektrum
($CDCl_3$) δ = 4,50 (s) (S–$CH_2$) 2 H, 6,97 (t) (Aromaten-H) 1 H, 7,13 (dd) (Aromaten-H) 1 H, 7,90 (d) (Aromaten-H) 1 H, 8,23 (d) (Aromaten-H) 1 H, 8,50 (d) (Aromaten-H) 2 H ppm.

Beispiel 11

Herstellung von 2-(Pyridyl-3-methylthio)-4,5-diphenyl-oxazol-Hydrochlorid

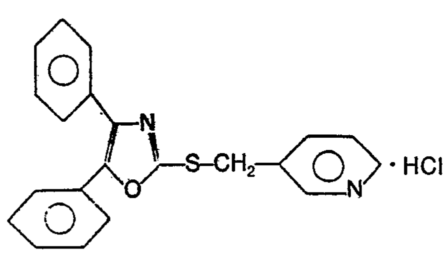

Die Herstellung erfolgt analog Beispiel 1 aus 2-Mercapto-4,5-diphenyloxazol und 3-Picolylchlorid-Hydrochlorid.
Farblose Kristalle vom Schmelzpunkt 156–157 °C
Rf = 0,62 ($CH_2Cl_2$ / MeOH 95:5 $NH_3$-Dämpfe)
Ausbeute: 17,9 g (47%)
$C_{21}H_{17}ClN_2OS$ (381)
Ber.: C 66,22 H 4,50 N 7,35
Gef.: C 66,23 H 4,50 N 7,47
$^1$H-NMR-Spektrum
($d_4$-MeOH): δ = 4,73 (s) (S–$CH_2$) 2 H, 7,20–7,67 (m) (Aromaten-H) 10 H, 8,13 (t) (Aromaten-H) 1 H, 8,73–9,0 (m) (Aromaten-H) 2 H, 9,10 (s) (Aromaten-H) 1 H ppm.

Beispiel 12

Herstellung von 2-(Pyridyl-3-methylthio)-1-methylimidazol-Dihydrochlorid

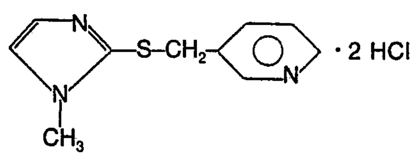

Die Herstellung erfolgt analog Beispiel 1 aus 2-Mercapto-1-methylimidazol und 3-Picolylchlorid-Hydrochlorid.
Farblose Kristalle vom Schmelzpunkt 187–189 °C
Rf = 0,43 ($CH_2Cl_2$ / MeOH 95:5 $NH_3$-Dämpfe)
Ausbeute: 12,0 g (43%)
$C_{10}H_{15}Cl_2N_3S$ (280)
Ber.: C 42,86 H 5,40 N 15,00
Gef.: C 43,08 H 4,77 N 15,03
$^1$H-NMR-Spektrum
($D_2O$): δ = 4,43 (s) (N–$CH_3$) 3 H, 5,13 (s) (S–$CH_2$) 2 H, 8,00–9,53 (m) (Aromaten-H) 6 H ppm.

Beispiel 13

Herstellung von 2-(Pyridyl-3-methylthio-6-chlor)-pyrimidin

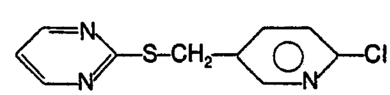

Die Herstellung erfolgt analog Beispiel 1 aus 2-Mercaptopyrimidin und 6-Chlor-3-picolylchlorid-Hydrochlorid.

Farblose Kristalle vom Schmelzpunkt 89–90 °C
Rf = 0,68 ($CH_2Cl_2$ / MeOH 9:1)
Ausbeute: 10,0 g (42%)
$C_{10}H_8ClN_3S$ (238)
$^1$H-NMR-Spektrum
($CDCl_3$): δ = 4,33 (s) (S–$CH_2$) 2 H, 7,00 (t) (Aromaten-H) 1 H, 7,20 (d) (Aromaten-H) 1 H, 7,77 (dd) (Aromaten-H) 1 H, 8,50 (m) (Aromaten-H) 3 H ppm.

Beispiel 14
Herstellung von 4-(Pyridyl-3-methylthio) 1 H-pyrazolo-(3,4-d)-pyrimidin

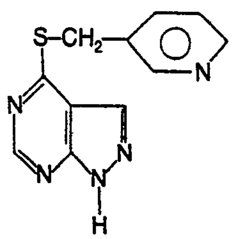

Die Herstellung erfolgt analog Beispiel 1 aus 4-Mercapto-1-H-pyrazolo-(3,4-d)-pyrimidin und 3-Picolylchlorid-Hydrochlorid.
Farblose Kristalle vom Schmelzpunkt 148–149 °C
Rf = 0,54 ($CH_2Cl_2$ / MeOH 9:1)
Ausbeute: 16,0 g (66%)
$C_{11}H_9N_5S$ (243)

| | | | |
|---|---|---|---|
| Ber.: | C 54,31 | H 3,73 | N 28,79 |
| Gef.: | C 54,27 | H 3,77 | N 28,75 |

$^1$H-NMR-Spektrum
($d_6$-DMSO): δ = 4,70 (s) (S–$CH_2$) 2 H, 7,33 (dd) (Aromaten-H) 1 H, 8,27 (s) (Aromaten-H) 1 H, 8,50 (d) (Aromaten-H) 1 H, 8,73 (s) (Aromaten-H) 1 H, 8,83 (s) (Aromaten-H) 1 H, 14,13 (s) (N–H) 1 H (austauschbar mit $D_2O$) ppm.

Beispiel 15
Herstellung von den erfindungsgemässen Wirkstoff enthaltenden Weichgelatinekapseln
(z. B. 2-(Pyridyl-3-methylthio)-pyrimidin-Hydrochlorid)
Zusammensetzung:

| | |
|---|---|
| 2-(Pyridyl-3-methylthio)pyrimidin-Hydrochlorid | 100,0 mg |
| Rüböl | 281,0 mg |
| Bienenwachs | 2,0 mg |
| Partiell hydriertes Pflanzenöl | 8,0 mg |
| Sojalecithin | 8,0 mg |
| 3-Ethoxy-4-hydroxy-benzaldehyd | 1,0 mg |
| Gesamtfüllgewicht einer Kapsel | 400,0 mg |

Die Substanzen werden gemischt, homogenisiert und in üblicher Weise zu Weichgelatinekapseln verarbeitet.

Beispiel 16
Herstellung von den erfindungsgemässen Wirkstoff enthaltenden Dosier-Aerosol
(z. B. 2-(Pyridyl-3-methylthio)pyrimidin-Hydrochlorid)
Zusammensetzung:

| | |
|---|---|
| 2-(Pyridyl-3-methylthio)pyrimidin-Hydrochlorid | 10,0 mg |
| Sorbitantrioleat | 0,5 mg |
| Difluormethan | 35,5 mg |
| Dichlortetrafluorethan | 25,0 mg |
| Gesamtdosis pro Hub | 71,0 mg |

Die Substanzen werden kalt gelöst und 10 g Lösung in eine geeignete Druckgasverpackung eingebracht.

Beispiel 17
Herstellung von den erfindungsgemässen Wirkstoff enthaltenden Ampullen
(z. B. 2-(Pyridyl-3-methylthio)-pyrimidin-Hydrochlorid)
Zusammensetzung:

| | |
|---|---|
| 2-(Pyridyl-3-methylthio)-pyrimidin-Hydrochlorid | 100,0 mg |
| Polyethylenglykol 300 | 630,0 mg |
| 1,2-Propandiol | 735,0 mg |
| α-Tocopherol | 1,0 mg |
| Dinatriumhydrogenphosphat | 4,0 mg |
| Natriumdihydrogenphosphat | 20,0 mg |
| Wasser für Injektionszwecke | 610,0 mg |
| Gesamtfüllgewicht | 2100,0 mg |

Die Substanzen werden gelöst und die Lösung in üblicher Weise zu Ampullen à 2 ml verarbeitet.

**Patentansprüche für die Vertragsstaaten BE, CH, DE, FR, GB, IT, LI, LU, NL, SE**

1. 3-Thiomethylpyridin-Derivate der allgemeinen Formel

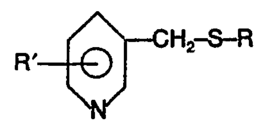

in der R die Gruppe 2-Pyrimidinyl, 2-(4-Methylpyrimidinyl), 2-(4,6-Dimethylpyrimidinyl), 2-Benzoxazolyl, 6-Methyl-2-benzoxazolyl, 2-Oxazolo-(4,5-c)-pyridinyl, 4-Tolyl, 4-Chlorphenyl, 2-(1-Methylimidazolyl), 2-(4,5-Diphenyloxazolyl) oder 4-(1H-Pyrazolo-(3,4-d)-pyrimidinyl) ist und R′ für ein Wasserstoffatom oder einen in 2- oder 6-Position angeordneten Methyl- oder Chlorrest steht, sowie ihre therapeutisch verträglichen Säureadditionssalze.

2. Verfahren zur Herstellung von 3-Thiomethylpyridin-Derivaten nach Anspruch 1, dadurch gekennzeichnet, dass man in an sich bekannter Weise ein Thiolat der allgemeinen Formel

R–SMe

in der R die in Anspruch 1 angegebene Bedeutung hat und Me für ein Alkaliatom steht, mit einem Picolylchlorid-Hydrohalogenid der Formel

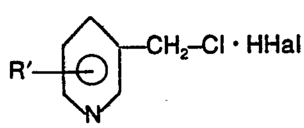

worin R′ wie in Anspruch 1 definiert ist und Hal ein Halogenatom bedeutet, in der wässrig-alkoholischen Alkalihydroxidlösung, wobei das Alkalihydroxid im Überschuss über die stöchiometrische Menge vorliegt, umsetzt und gegebenenfalls die

so erhaltene freie Base in ein therapeutisch verträgliches Säureadditionssalz umwandelt.

3. Pharmazeutische Zubereitung mit bronchosekretolytischer und mukolytischer Wirkung, enthaltend wenigstens eine Verbindung nach Anspruch 1 und übliche Hilfs- und Trägerstoffe.

**Patentanspruch für den Vertragsstaat AT**

Verfahren zur Herstellung von 3-Thiomethylpyridin-Derivaten der allgemeinen Formel

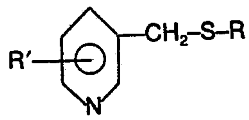

in der R die Gruppe 2-Pyrimidinyl, 2-(4-Methylpyrimidinyl), 2-(4,6-Dimethylpyrimidinyl), 2-Benzoxazolyl, 6-Methyl-2-benzoxazolyl, 2-Oxazolo-(4,5-c)-pyridinyl, 4-Tolyl, 4-Chlorphenyl, 2-(1-Methylimidazolyl), 2-(4,5-Diphenyloxazolyl) oder 4-(1H-Pyrazolo-(3,4-d)-pyrimidinyl) ist und R′ für ein Wasserstoffatom oder einen in 2- oder 6-Position angeordneten Methyl- oder Chlorrest steht, sowie ihrer therapeutisch verträglichen Säureadditionssalze, dadurch gekennzeichnet, dass man in an sich bekannter Weise ein Thiolat der allgemeinen Formel

R–SMe

in der R die oben angegebene Bedeutung hat und Me für ein Alkaliatom steht, mit einem Picolylchlorid-Hydrohalogenid der Formel

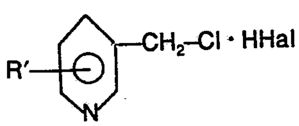

worin R′ wie oben definiert ist und Hal ein Halogenatom bedeutet, in der wässrig-alkoholischen Alkalihydroxidlösung, wobei das Alkalihydroxid im Überschuss über die stöchiometrische Menge vorliegt, umsetzt und gegebenenfalls die so erhaltene freie Base in ein therapeutisch verträgliches Säureadditionssalz umwandelt.

**Claims for the contracting States BE, CH, DE, FR, GB, IT, LI, LU, NL, SE**

1. 3-thiomethylpyridine derivatives corresponding to the following general formula

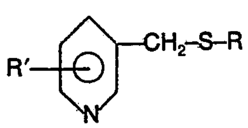

wherein R denotes the group 2-pyrimidinyl, 2-(4-methylpyrimidinyl), 2-(4,6-dimethylpyrimidinyl), 2-benzoxazolyl, 6-methyl-2-benzoxazolyl, 2-oxazolo-(4,5-c)-pyridinyl, 4-tolyl, 4-chlorophenyl, 2-(1-methylimidazolyl), 2-(4,5-diphenyloxazolyl) or 4-(1H-pyrazolo-(3,4-d)-pyrimidinyl) and R′ denotes a hydrogen atom or a methyl or chlorine group arranged in the 2- or 6-position, and their therapeutically acceptable acid addition salts.

2. Process for the preparation of 3-thiomethylpyridine derivatives according to Claim 1, characterised in that a thiolate corresponding to the general formula

R–SMe

wherein R has the meaning indicated in Claim 1 and Me stands for an alkali metal atom is reacted in known manner with a picolyl chloride hydrohalide corresponding to the formula

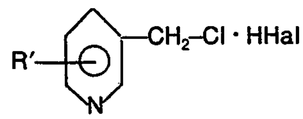

wherein R′ has the meanings defined in Claim 1 and Hal denotes a halogen atom in the aqueous-alcoholic alkali metal hydroxide solution, the alkali metal hydroxide being present in excess of the stoichiometric quantity and the resulting free base is optionally converted into a therapeutically acceptable acid addition salt.

3. Pharmaceutical preparation having a bronchosecretolytic and mucolytic action, containing at least one compound according to Claim 1 and conventional auxiliary agents and carriers.

**Claim for the contracting State: AT**

Process for the preparation of 3-thiomethylpyridine derivatives corresponding to the following general formula

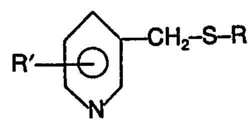

wherein R denotes the group 2-pyrimidinyl, 2-(4-methylpyrimidinyl), 2-(4,6-dimethylpyrimidinyl), 2-benzoxazolyl, 6-methyl-2-benzoxazolyl, 2-oxazolo-(4,5-c)-pyridinyl, 4-tolyl, 4-chlorophenyl, 2-(1-methylimidazolyl), 2-(4,5-diphenyloxazolyl) or 4-(1H-pyrazolo-(3,4-d)-pyrimidinyl) and R′ denotes a hydrogen atom or a methyl or chlorine group which is arranged in the 2- or 6-position, and therapeutically acceptable acid addition salts thereof, characterised in that a thiolate corresponding to the general formula

R–SMe

wherein R has the meaning indicated above and Me stands for an alkali metal atom is reacted in known manner with a picolyl chloride hydrohalide corresponding to the formula

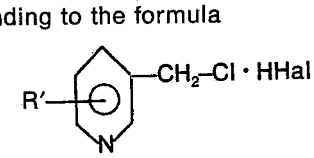

wherein R′ has the meanings defined above and Hal denotes a halogen atom in the aqueous-alcoholic alkali metal hydroxide solution, the alkali

metal hydroxide being present in excess of the stoichiometric quantity and the resulting free base is optionally converted into a therapeutically acceptable acid addition salt.

**Revendications pour les Etats contractants: BE, CH, DE, FR, GB, IT, LI, LU, NL, SE**

1. Dérivés de 3-thiométhylpyridine de formule générale

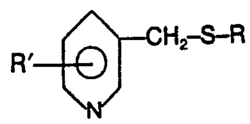

dans laquelle R représente le groupe 2-pyrimidinyle, 2-(4-méthylpyrimidinyle), 2-(4,6-diméthylpyrimidinyle), 2-benzoxazolyle, 6-méthyl-2-benzoxazolyle, 2-oxazolo-(4,5-c)-pyridinyle, 4-tolyle, 4-chlorophényle, 2-(1-méthylimidazolyle), 2-(4,5-diphényloxazolyle) ou 4-(1H-pyrazolo-(3,4-d)-pyrimidinyle) et R′ représente un atome d'hydrogène ou un radical méthyle ou chloro situé en position 2 ou 6, ainsi que leurs sels d'addition d'acides thérapeutiquement acceptables.

2. Procédé pour la préparation des dérivés de 3-thiométhylpyridine selon la Revendication 1, caractérisé en ce que l'on fait réagir d'une manière connue en soi un thiolate de formule générale

R–SMe

dans laquelle R est défini comme spécifié dans la Revendication 1 et Me représente un atome alcalin, avec un hydracide halogéné d'un hydrohalogénure de chlorure de picolyle de formule

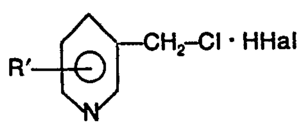

dans laquelle R′ est défini comme spécifié dans la Revendication 1 et Hal désigne un atome d'halogène, dans la solution hydro-alcoolique d'un hydroxyde alcalin, l'hydroxyde alcalin étant présent en excès par rapport à la quantité stoechiométrique, et on transforme éventuellement la base libre ainsi obtenue en un sel d'addition d'acide thérapeutiquement acceptable.

3. Composition pharmaceutique à activité bronchosécrétolytique et mucolytique, contenant au moins un composé selon la Revendication 1 et des adjuvants et véhicules courants.

**Revendication pour l'Etat contractant: AT**

Procédé pour la préparation de dérivés de 3-thiométhylpyridine de formule générale

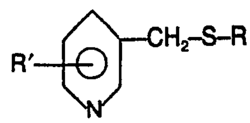

dans laquelle R représente le groupe 2-pyrimidinyle, 2-(4-méthylpyrimidinyle), 2-(4,6-diméthylpyrimidinyle), 2-benzoxazolyle, 6-méthyl-2-benzoxazolyle, 2-oxazolo-(4,5-c)-pyridinyle, 4-tolyle, 4-chlorophényle, 2-(1-méthylimidazolyle), 2-(4,5-diphényloxazolyle) ou 4-(1H-pyrazolo-(3,4-d)-pyrimidinyle) et R′ représente un atome d'hydrogène ou un radical méthyle ou chloro situé en position 2 ou 6, ainsi que leurs sels d'addition d'acides thérapeutiquement acceptables, caractérisé en ce que l'on fait réagir d'une manière connue en soi un thiolate de formule générale

R–SMe

dans laquelle R est défini comme spécifié ci-dessus et Me représente un atome d'un alcalin, avec un hydracide halogéné d'un hydrohalogenure de chlorure de picolyle de formule

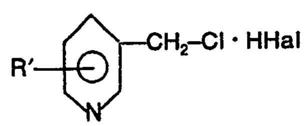

dans laquelle R′ est défini comme spécifié ci-dessus et Hal désigne un atome d'halogène, dans la solution hydro-alcoolique d'un hydroxyde alcalin, d'un hydroxyde alcalin, l'hydroxyde alcalin étant présent en excès par rapport à la quantité stoechiométrique, et on transforme éventuellement la base libre ainsi obtenue en un sel d'addition d'acide thérapeutiquement acceptable.